# EUROPEAN PATENT SPECIFICATION

(11) **EP 3 305 370 B1**
(45) Date of publication and mention of the grant of the patent: **10.03.2021**
(21) Application number: 16192214.1
(22) Date of filing: 04.10.2016
(51) Int. Cl.: A61Q 19/08, A61K 8/97

(54) **ALGAE AUTOPHAGY ACTIVATOR**
ALGENAUTOPHAGIEAKTIVATOR
ACTIVATEUR D'AUTOPHAGIE DES ALGUES

(43) Date of publication of application: 11.04.2018
(73) Proprietor: Chemisches Laboratorium Dr. Kurt Richter GmbH, 12277 Berlin (DE)
(72) Inventor: Hofmeister, Dirk, 12277 Berlin (DE); John, Sabrina, 12277 Berlin (DE); Bittroff, Sebastian, 12277 Berlin (DE); Prade, Heiko, 12277 Berlin (DE); Van der Hoeven, Harald, 12277 Berlin (DE)
(74) Representative: Rupp, Christian

(56) References cited:
- WO-A1-2016/030643
- M Bimonte ET AL: "Galdieria sulphuraria Relieves Oily and Seborrheic Skin By Inhibiting the 5-[alpha] Reductase Expression in Skin Cells and Reducing Sebum Production In Vivo", Trichology and Cosmetology, 17 May 2016 (2016-05-17), pages 1-8, XP055338860, DOI: 10.17140/TCOJ-1-103 Retrieved from the Internet: URL:http://openventio.org/Volume1_Issue1/G aldieria_sulphuraria_Relieves_Oily_and_Seb orrheic_Skin_By_Inhibiting_the_5_Reductase _Expression_in_Skin_Cells_and_Reducing_Seb um_Production_In_Vivo_TCOJ_1_103.pdf [retrieved on 2017-01-25]
- MYOUNGHOON MOON ET AL: "Isolation and characterization of thermostable phycocyanin from Galdieria sulphuraria", KOREAN JOURNAL OF CHEMICAL ENGINEERING, vol. 31, no. 3, 15 January 2014 (2014-01-15), pages 490-495, XP055146911, ISSN: 0256-1115, DOI: 10.1007/s11814-013-0239-9
- BERMEJO PALOMA ET AL: "Iron-chelating ability and antioxidant properties of phycocyanin isolated from a protean extract ofSpirulinaplatensis", FOOD CHEMISTRY, vol. 110, no. 2, 2008, - 20 August 2000 (2000-08-20), pages 436-445, XP029235778, ISSN: 0308-8146, DOI: 10.1016/J.FOODCHEM.2008.02.021
- LAILA SØRENSEN ET AL: "Purification of the photosynthetic pigment C-phycocyanin from heterotrophic Galdieria sulphuraria", JOURNAL OF THE SCIENCE OF FOOD AND AGRICULTURE, vol. 93, no. 12, 5 September 2013 (2013-09-05), pages 2933-2938, XP055324284, GB ISSN: 0022-5142, DOI: 10.1002/jsfa.6116
- DATABASE GNPD [Online] MINTEL; July 2016 (2016-07), "Correcting cream", XP002766455, Database accession no. 4063653
- Mengqiang Li ET AL: "Inhibition of androgen induces autophagy in benign prostate epithelial cells : Finasteride provokes autophagy in BPH", INTERNATIONAL JOURNAL OF UROLOGY., vol. 21, no. 2, 1 February 2014 (2014-02-01), pages 195-199, XP055673934, JP ISSN: 0919-8172, DOI: 10.1111/iju.12210

## Description

The present invention relates to microalgae extracts, particularly extracts from *Galdieria* sp., their preparation and their use in cosmetics, particularly for topic use in skin and hair care. The invention further relates to an autophagy activator and an iron-binding activity from *Galdieria* sp.. In particular, the invention relates to uses of such extracts, the autophagy activator and/or the iron-binding activity for improving age-related skin appearance, or for cosmetic treatment and prevention of aged skin, such as photo-aged skin.

### Background of the invention

The average age of the society is steadily rising so that the symptoms of aging will influence our daily life to a wider extent. Nowadays, it is discussed if the term aging could be regarded as an illness (Bulterijs et al., 2015). Different molecular biological pathways related to aging and theories in this respect are proposed. López-Otin et al. (2013) summarized hallmarks for aging that are commonly accepted: genomic instability, telomere attrition, epigenetic alterations, loss of proteostasis, deregulated nutrient sensing, mitochondrial dysfunction, cellular senescence, stem cell exhaustion, and altered intercellular communication.

### Lysosomal aging

The theory of lysosomal aging is based on the idea of a steady turnover, which is disturbed by the malfunction of the endosomal/lysosomal regeneration unit (Brunk and Terman, 2002; Cuervo and Dice, 2000). During cell aging the total amount of iron rises steadily. The increase in total iron is also dependent on the metabolic activity of the cell because oxygen is provided via haemoglobin, which contains iron as the oxygen acceptor. The higher the metabolic activity, the more educts are needed and in consequence more iron is delivered to the cell. Only a limited number of cells are able to transport iron out of the cell actively with the help of ferroportin. Examples for these cells are hepatocytes, macrophages, enterocytes, and adipocytes.

The lysosome is an organelle, which derives from the Golgi apparatus and was first discovered by serendipity by Christian de Duve in the early 1950s. In a premature state, they are so-called early endosomes. In this state, the internal pH is already lowered to 6. During maturation, the degrading enzymes, e.g. lipases and hydrolases, become more active, and the pH finally decreases to 4.5-5 due to an ATP-driven H⁺ pump. Certain publications indicate that different kinds of lysosomes exist. The specialization is characterised via size and function. Next to the cellular turnover and recycling, the functions of lysosomes are also associated with the energy status and the related longevity of the cell, the communication between cells and the repair of damaged membrane (Settembre et al., 2013; Xu and Ren, 2015). Rupar et al. (1992) found that the specific content of alpha-tocopherol in rat liver lysosomes is 37 times higher than in other cellular membranes.

Therefore, the maintenance and protection of lysosomal activity is considered to support the correct functions of these processes and could substantially retard cell aging via an improvement of either the quantity or the quality of basic molecules, e.g. collagen, elastin, cholesterol, ceramides, and sphingolipids.

Based on previous research it is assumed that the increase of reactive oxygen species (ROS) in lysosomes leads to defective autophagosomes (Brunk and Terman, 2002; Kurz et al., 2011). This process depends on the Fenton reaction and is therefore iron dependent. The higher the iron concentration per lysosome, the higher the probability that a Fenton reaction proceeds, which results in a permeabilization of the surrounding membrane (Kurz et al., 2011). On this basis, one can conclude that it is favourable for the single cell to regulate its total amount of iron in relation to its metabolic activity. Only a limited number of cells are capable of an active iron export (Baird et al., 2006). Therefore, the use of iron chelators, e.g. desferrioxamine (DFO), will prevent or lower the lipid peroxidation and the resulting permeabilization, if they are present during the lysomal degradation of iron containing molecules like ferritin (De Domenico et al., 2009; Kurz et al., 2011). Unfortunately, DFO is retained in lysosomes and finally leads to cell death (Kurz et al., 2008).

Generation of additional lysosomes represents another possibility to decrease the iron content per lysosomes. Finally, the temporary and therefore milder chelation of iron inside the lysosomes reduces the ROS associated damages while not reducing the amount of H₂O₂ (Baird et al., 2006; De Domenico et al., 2009).

Fransen et al. (2012) stated that no convincing experimental evidence supports the postulated theory that the mitochondria is the primary source of ROS. During the differentiation of the keratinocytes the contribution of mitochondrial generated ROS is estimated substantial only in the stem cells of the basal layer (Rinnerthaler et al., 2015). Therefore, the energy intensive process of the differentiation has to be supported by other sources. The peroxisomes and the lysosomes could deliver the necessary energy and the building blocks for the anabolism of the macromolecules, e.g. involucrin, keratin, loricrin, and profilaggrin.

### Ferroptosis

Ferroptosis is a non-apoptotic form of cell death that is iron dependent (Dixon et al., 2012). Recently, it has been shown that during ferroptosis lipid peroxidation is accompanied by lethal ROS production, which could be prevented via the use of iron chelators, e.g. deferoxamine (Xie et al., 2016). Additionally, a relationship between autophagy and ferroptosis has been found (Hou et al., 2016).

### Autophagy

Autophagy plays an important role in many physiological and pathological processes. In the absence of stress, autophagy serves a house keeping function, removing damaged organelles and cellular components, thus preventing cytotoxic effects. Decreases and defects in autophagy have been implicated in multiple diseases, for example Huntington's, Alzheimer's, and Parkinson's disease. On the other hand, the relevance of autophagy as a detoxifying mechanism, which can promote health and longevity has been shown (Pernodet et al., 2014; Rubinsztein et al., 2011).

Microtubule-associated protein 1A/1B-light chain B (LC3B) is a widely accepted marker of autophagy. Pernodet et al. (2016) found a difference of 77.9% between the LC3B level in fibroblasts of young and mature donors, suggesting that LC3B is also a marker of age-related functional losses of the cell. Akinduro et al. (2016) found a strong relationship between autophagy-linked WIPI-1 and the expression of LC3B in the epidermis during differentiation.

WO 2014/092859 discloses compositions for topical application to the skin comprising a WIPI-1 agonist and the use of such compositions to provide benefits to the skin, in particular, an improvement of autophagy activity within skin cells and thus improvement of the condition and appearance of chronologically or photo-aged skin. Such a WIPI-1 agonist is contained, for example, in extracts of *Tiliacora triandra,* a species of flowering plant.

WO 2010/063977 discloses the cosmetic use of skin cell autophagy activators.

### Lysosomal activity in different skin compartments

### Stratum basale

The stratum basale inherits the epidermal stem cells that proliferate to enable the regeneration of the differentiating keratinocytes. The main function of the lysosomes in this part of the epidermis is the elimination of toxic cellular waste, e.g. misfolded proteins.

### Stratum spinosum

In this part of the epidermis the degradation of the cell organelles, e.g. the nucleus or mitochondria is started. There are different possible positive effects. Since further differentiation is an energy consuming process in the first place, alternative supply is needed with decreasing mitochondrial activity. Lysosomal degradation is able to deliver acetyl-CoA and glycerol-3-phosphate. At this level the cells are still communicating intercellularly and do need endocytosis and exocytosis as a carrier mechanism for signal molecules such as the endocytosis of melanosomes.

### Stratum granulosum

The role of the lysosomes in this part of the epidermis is under-estimated and not well described. We propose that especially during the differentiation of the keratinocytes to corneocytes lysosomes play a major role. The lipophilic barrier function, the epidermal pH and Ca²⁺ gradient are crucial for a proper physical function and could be influenced by lysosomal membrane stability (Raymond et al., 2008). Lysosomes are able to build up a 5,000-fold Ca²⁺ gradient between lysosomal lumen and cytosol (Xu and Ren, 2015). The build-up and the status of a calcium gradient is only explainable by a recycling or retention mechanism (Elias et al., 2002).

### Stratum lucidum

The nearly completely differentiated keratinocyte passes the stratum lucidum, while secreting the necessary lipohilic substances to maintain the hydrophobic barrier. The vesicles where the lipophilic substances are incorporated are called Odland or lamellar bodies. Recently they have been considered to be "lysosome related organelles" because luminal and membrane related proteins like LAMP1 have been discovered inside these organelles (Raymond et al., 2008).

### Object of the invention

WO 2013/023873 discloses aqueous extracts from *Cyanidium caldarium* (Rhodophyta, Cyanidales) containing 4-amino butyric acid (GABA) for cosmetic applications. *Cyanidium caldarium* has been reported to synthetize spermine and spermidine, which are capable of increasing autophagy (Pietrocola et al., 2015).

WO 2014/043009 discloses a skin care composition for improving selective catabolysis in cells of keratin surfaces comprising a cellular autophagy activator and a cellular proteasome activator WO2016030643 discloses extracts from Arthrospira platensis enriched in phycobiliproteins such as phycocyanin and their use in anti-wrinkle cosmetic compositions.

Bimonte et al. (2016) found a water-soluble extract of *Galdieria sulphuraria* to be capable of inhibiting 5-α reductase, inducing the expression of the β-defensins, which represents the first defense response mechanism triggered by the skin cells to fight against undesired microbes, and stimulating the wound healing process in skin cell cultures. According to the authors, their results suggested that *Galdieria* extract had interesting potentialities as active ingredient in cosmetic and dermatological formulas, in particular in those addressed to oily and seborrheic skins. *Galieria* sp. also expresses spermidine (Seckbach, 1994).

There is, however, a need for further natural sources of activators of cellular defense against skin damage, which sources are readily available, can be processed at large-scale and are appropriate for cosmetic purposes.

### Summary of the invention

The object of the invention is achieved by providing *Galdieria* sp. and extracts thereof containing activities capable of stimulating lysosomal turnover and autophagy in keratinocytes.

In one aspect, the invention provides an extract from *Galdieria sulphuraria,* comprising an autophagy activator and/or an iron-binding agent, or an autophagy activator having iron binding properties.

In one aspect, there is provided a cosmetic and/or non-pharmaceutical composition comprising or consisting of an extract according to the invention.

In one aspect, the invention provides a process of preparation of an autophagy activator and/or an iron-binding agent, or an autophagy activator having iron binding properties, or an extract or a composition, comprising the steps of:
(a) Providing a cell culture of *Galdieria* sp., preferably *Galdieria sulphuraria;*
(b) Harvesting the cultured cells provided in step (a);
(c) Disrupting the cultured cells harvested in step (b); and
(d) Obtaining an extract comprising the cells disrupted in step (c).

In one aspect, there is provided an autophagy activator and/or an iron-binding agent, or an autophagy activator having iron binding properties, or an extract or a composition obtained in the process of preparation according to the invention.

In one aspect, there is provided a cosmetic and/or non-pharmaceutical use of an autophagy activator and/or an iron-binding agent, or an autophagy activator having iron binding properties, or an extract or a cell preparation or a composition according to the invention for topical, extradermal or exogenous application to the skin or hair of a subject.

In one aspect, there is provided a cosmetic and/or non-pharmaceutical, use of an autophagy activator and/or an iron-binding agent, or an autophagy activator having iron binding properties, or an extract or a cell preparation or a composition according to the invention for stimulating or increasing lysosomal turnover and/or activity in skin cells, preferably keratinocytes, in a subject.

In one aspect, there is provided a cosmetic and/or non-pharmaceutical use of an autophagy activator and/or an iron-binding agent, or an autophagy activator having iron binding properties, or an extract or a cell preparation or a composition according to the invention for stimulating or increasing autophagy in skin cells, preferably keratinocytes, in a subject.

In one aspect, there is provided a cosmetic and/or non-pharmaceutical use, or a pharmaceutical or therapeutic use of an autophagy activator and/or an iron-binding agent, or an autophagy activator having iron binding properties, or an extract or a cell preparation or a composition according to the invention in a subject supposed to benefit from increased lysosomal turnover and/or activity, and/or autophagy.

In one aspect, there is provided a cosmetic and/or non-pharmaceutical use of an autophagy activator and/or an iron-binding agent, or an autophagy activator having iron binding properties, or an extract or a cell preparation or a composition according to the invention for retarding the development, preferably an accelerated development, or the manifestation, preferably a premature manifestation of aged skin.

In one aspect, there is provided a cosmetic and/or non-pharmaceutical use of an autophagy activator and/or an iron-binding agent, or an autophagy activator having iron binding properties, or an extract or a cell preparation or a composition according to the invention for treating or preventing signs of altered or aged skin, or for treating or preventing photo-aging in a subject.

In one aspect, there is provided a cosmetic and/or non-pharmaceutical use of an autophagy activator and/or an iron-binding agent, or an autophagy activator having iron binding properties, or an extract or a cell preparation or a composition according to the invention for improving age-related skin appearance in a subject.

In yet further aspects, the invention provides methods of use or methods of treatment referring or relating to any other aspects or embodiments of the invention.

### Detailed description of the invention

The present invention features extracts from microalga *Galdieria* sp., i.e. a species of unicellular red algae (Rhodophyta, Cyanidales, Galdieriaceae). The organism is polyextremophilic, for example grows at temperatures up to 56°C and tolerates pH values of less than 2 (Gross et al., 1998; Jain et al., 2014). Near relatives are, for example, *Cyanidioschyzon merolae* and *Cyanidium caldarium.*

The invention is based on the findings that *Galdieria* extract increases lysosomal activity and LC3B protein in cultured keratinocytes. These findings indicate that *Galdieria* extract stimulates autophagy in skin cells and thus contains one or more autophagy activators. The *Galdieria* extract furthermore displays iron binding properties assumed to aid in autophagy.

The *Galdieria* extract or cosmetic compositions containing the same are useful for personal skin and hair care. An advantage of the extract or compositions is the stimulation of cellular detoxification. In doing so, the extract or compositions protect human skin against various types of damage, both from the inside and from the outside (e.g. through sunlight), or help alleviate signs of such damage. Thereby, the extract or compositions improve the overall aesthetic appearance of the skin, in particular of aged skin.

Another advantage of the *Galdieria* extract is its biological source. This usually enhances the acceptance of consumers and may be a condition for use in natural or organic cosmetic compositions.

Yet other advantages are that stock cultures of *Galdieria* sp. are commercially available; great quantities of *Galdieria* biomass are producible using ordinary procedures at large-scale; the processing of biomass is rarely associated with particular, e.g. toxic risks for the staff; and the extracts are readily storable, e.g. after lyophilisation.

### Explanations and definitions

Generally, "autophagy" or "autophagocytosis" describes a homeostatic process for removing from the cell, for example, dysfunctional cellular components, misfolded proteins, damaged organelles, or intracellular microbial pathogens. In this way, autophagy serves the functions of cell detoxification and defense against cell damage. There are three pathways of autophagy, namely macroautophagy, microautophagy and chaperone-mediated autophagy. The main pathway, macroautophagy, involves the formation of a double membrane known as an autophagosome around the component marked for destruction. The autophagosome then travels through the cytoplasm of the cell to a lysosome, and the two organelles fuse. Within the lysosome, the contents of the autophagosome are degraded via acidic lysosomal hydrolases.

A "lysosome" is a membrane-bound vesicle containing hydrolytic enzymes in the production of which ribosomes, endoplasmatic recticulum and Golgi apparatus are involved.

An "endosome" is a membrane-bound compartment containing, for example, cellular material to be degraded, which eventually fuses with a lysosome.

The description *"Galdieria* sp." means *Galdieria sulphuraria.*

Several "autophagy activators" are known in the art. Since different types of signalling pathways are involved, autophagy activators may intervene in the process at different stages. For example, rapamycin, everolimus, resveratrol and tamoxifen act as mTOR inhibitors.

An "iron chelating agent" or iron chelator binds iron through chelate complex formation, i.e. in a non-covalent manner.

As used herein, the term "iron-binding agent" also means "iron-binding activity".

The term "extract" as used herein refers to a preparation of algal cells after having been subjected to at least one cell disruption treatment. An extract may contain from at least 60% to 80%, 90%, 95% or more disrupted cells. An extract is prepared by ultra-sonification, high-pressure homogenisation or freezing/thawing of a cell suspension and contains cell debris. The term thus includes preparations from crude homogenates or lysates up to highly purified fractions thereof. The term also includes filtrates obtained from homogenates or lysates or fractons thereof. The term furthermore includes reconstitutes, e.g. resuspensions or resolutions, of previously lyophilised or otherwise dried extracts.

The term "cell suspension" as used herein refers to a preparation of algal cells not having been subjected to any cell disruption treatment. For examples, a cell suspension contains from at least 50% to 80%, 90%, 95% or more intact cells. The term intends to lay the focus on cells not disrupted on purpose, irrespective of the fact that, for example, a cell suspension treated by ultra-sonification contains cell debris and thus, from a pure physical perspective, may qualify as a suspension.

The term "active ingredient" or "active agent" as used herein refers to an ingredient or agent displaying autophagy and/or iron-binding activity.

The term "aging" as used herein refers to the naturally and irreversibly occurring alterations in biological processes of cells and organisms over time (chronological aging).

The term "aged skin" as used herein refers to optically or otherwise identifiable alterations of the skin including, for example, lines, wrinkles, pigmented spots, liver spots, solar lentigines, age spots, spider veins, senile warts, and parchment skin. The term includes skin appearance due to chronological, i.e. "physiological" or "normal" or "adequate" aging. The term also considers accelerated or premature aging, i.e. aged skin appearance due to, for example, extensive exposure to sun light (e.g. chronic UVA and UVB exposure), smoking, alcohol consumption, drug abuse, or inadequate nutrition or skin care.

The term "altered skin" as used herein refers to the appearance of the skin of a subject, which deviates from a former appearance of the skin, for example at a time when the subject was younger or not yet suffering from a skin disorder or disease.

The term "photo-aged skin" relates to skin showing signs of photo-aging (also referred to as dermatoheliosis) such as wrinkles, solar lentigines, age spots, or leathery skin appearance.

The term "lysosome related aged skin" as used herein relates to reduced or otherwise affected lysosome activity or turnover in skin cells, for example in contrast to impaired elastine or collagen function.

The term "lysosome activity" as used herein includes lysosome turnover.

### Preferred embodiments of the invention

In one embodiment, *Galdieria sulphuraria* is or includes a subspecies of *Galdieria sulphuraria* (i.e. *Galdieria sulphuraria* ssp.) In one embodiment, the autophagy activator and/or the iron-binding agent is an organic molecule or compound. In another embodiment, the autophagy activator and/or the iron-binding agent is an inorganic molecule or compound.

In one embodiment, the autophagy activator is a protein or a protein complex or a proteinaceous complex. For example, the autophagy activator binds to pattern recognition receptors (PRR) such as Toll-like receptors (TLR) or Nod like receptors. For example, signal transduction induced by the autophagy activator involves chaperones or heat shock proteins like Hsp70.

In one embodiment, the autophagy activator is not or excludes spermine or spermidine. In another embodiment, the autophagy activator is not dependent on or related to spermine or spermindine. For example, the autophagy activator and spermine or spermidine do not share a common cellular receptor or do not share all steps of the signalling pathway.

In one embodiment, the iron-binding agent is an iron-chelating agent. Alternatively, the iron-binding agent is a protein or protein complex.

In one embodiment, the autophagy activity and the iron-binding activity are located at different molecules, compounds, complexes, unities or cell components or compartments. In another embodiment, the autophagy activating activity and the iron-binding activity are located at the same molecule, compound, complex, unity or cell component or compartment.

In one embodiment, the autophagy activator and/or the iron-binding agent is isolated or purified or roughly purified or semi-purified from *Galdieria* extract.

In one embodiment, the autophagy activator and/or the iron-binding agent is the active ingredient, e.g. the only active ingredient, in a cosmetic and/or non-pharmaceutical composition, or in a pharmaceutical or therapeutic composition.

In one embodiment, the extract is prepared from cultured cells of *Galdieria sulphuraria.*

In one embodiment, the cell preparation is a cell suspension of the cultured cells. The cell preparation predominantly contains intact cells or whole cells or cells not having been subjected to a disruption or disintegration step, such as ultra-sonification. For example, the cell preparation contains culture medium in which the cells have been propagated, i.e. represents a liquid cell culture with the cells not having been subjected to a separation or washing step. In an alternative example, the cells have been harvested by a separation step, e.g. by centrifugation, and re-suspended in e.g. fresh culture medium, physiological saline, a buffered salt solution or water. Preferably, the cell preparation is an aliquot a liquid cell culture.

In one embodiment, the extract is preferred to the cell preparation.

In one embodiment, the cultured cells are preferably liquid cultured cells, i.e. have been propagated in liquid culture.

In one embodiment, the cultured cells are autotrophic or phototrophic cultured cells, preferably autotrophic cultured cells.

In one embodiment, the cultured cells have been harvested by separation from liquid culture medium using centrifugation, filtration, e.g. ultra-filtration, sedimentation or flotation. Preferably, separation is effected by centrifugation of the cell culture suspension, e.g. at 4,000 rpm, 2,800 x g for 10 minutes. Optionally, the cells have been washed with physiological saline or a buffered salt solution, e.g. once, twice, tree times, or several times.

In one embodiment, the extract is or is prepared from a cell homogenate, e.g. a crude homogenate, or a cell lysate, e.g. a crude lysate. The homogenate or lysate is prepared by disintegration or disruption of intact cells, e.g. by mechanical, chemical or thermal means. For example, the homogenate or lysate is prepared by freezing and thawing, or repeated freezing/thawing cycles; by means of osmolytic shock; by exposure to increased or lowered pressure, e.g. high pressure homogenisation; by blending, e.g. using a bead mill, ball mill, homogenizer or extruder; by ultra-sonification; or by means of surfactants, detergents, chaotropic salts, or cytolytic enzymes. Preferably, the extract is a crude cell lysate prepared by freezing and thawing of cells, more preferably by three freezing/thawing cycles. Preferably, the extract is a crude cell homogenate prepared by ultra-sonification of cells, most preferably by treating cells with ultrasound for 30 seconds, followed by 10 seconds of cooling (six cycles) at 200 W.

In one embodiment, the extract is a fraction of a cell homogenate or lysate, e.g. a particulate fraction, a pellet, a re-suspended pellet or an extract from a pellet. For example, the particulate fraction comprises outer membranes, intracellular membranes or organelles of cells.

In one embodiment, the extract is a filtrate obtained from a cell homogenate or lysate.

In one embodiment, the extract is a purified or roughly purified or semi-purified *Galdieria* extract.

In one embodiment, the extract is prepared using organic extraction protocols or solid purification means, e.g. a solid phase column.

In one embodiment, the extract is an aqueous or non-aqueous extract. In another embodiment, the extract is a mixture of aqueous and non-aqueous extract. For example, the non-aqueous extract is an organic extract, and is miscible or immiscible with water.

In one embodiment, the extract or cell preparation further contains an additive, which, for example, stabilizes the activity of the autophagy activator and/or iron-binding agent. For example, the additive is a preservative.

In one embodiment, the extract or cell preparation underwent freezing, drying or freeze-drying (lyophilisation). For example, the drying or freeze-drying is effected by means of spray drying or using a belt drier. For example, the extract or cell preparation is applied to a support or carrier for drying.

In one embodiment, the extract or cell preparation is a reconstitute, e.g. a resuspension or resolution, of a dried or freeze-dried extract.

In another embodiment, the extract or cell preparation is freshly prepared, i.e. did not undergo freezing, drying or freeze-drying.

In one embodiment, the composition is for exogenous, extradermal and/or cutaneous application. For example, the composition is for topical application to the skin and/or hair.

In one embodiment, the composition is from fluid to viscous, creamy, oily, powdery, gel-like, or solid.

In one embodiment, the composition is adapted to the administration by cutaneous topical means, e.g. a spatula, an ampoule, a vial, an applicator, a syringe, a tube, a jar, or a flask.

In one embodiment, the composition is a skin care and/or a hair care composition.

In one embodiment, the composition is an emulsion, for example an oil-in-water (o/w) or water-in-oil (w/o) emulsion, a multiple emulsion (water/oil/water or oil/water/oil), a microemulsion, a nanoemulsion, a pickering emulsion (i.e. stabilized by solid particles such as colloidal silica). For example, the emulsion is a non-ionic emulsion, a cationic emulsion or an anionic emulsion.

In one embodiment, the composition is a solution, a suspension, a hydrodispersion, an aqueous gel, an emulsified gel, an aerosol, or a loose or pressed powder.

In one embodiment, the composition is selected from the group consisting of a cream, a lotion, a milk, a paste, an ointment, a fluid, a liquid, a serum, a mousse, a foam, a gel, an oil, a powder, and a spray.

In one embodiment, the composition is selected from the group consisting of a skin cream, a body lotion, a body milk, a skin gel, a body spray, a foundation, a moisturizing after sun cooling gel, a face cleanser mask, a face defend cream gel, a hand care cream, a protecting foot care cream, a nappy rash cream, an anti-aging foundation, an anti-aging cream, a lipstick composition, a mascara composition, an eyeshadow composition, a skin care powder, a skin protecting body spray, a sun protection spray, a shampoo, a conditioner, a hair spray, and a hair dye product or colourant.

In one embodiment, the composition contains from about 0.01 to 20% (by weight), preferably from about 0.05% to 10%, more preferably from about 0.08% to 8%, or 0.1% to 5%, or 0.1% to 1.0%, or 0.1% to 0.5%, most preferably 0.3% of the extract or cell preparation.

In one embodiment, the composition contains, in addition to the extract, cell preparation or active ingredient, a cosmetically and/or pharmaceutically acceptable component, additive, adjuvant or vehicle. Such components do not cause a feeling of discomfort by the user such a flushing, tingling or tickling. For example, the composition contains a cosmetically and/or pharmaceutically acceptable base.

In one embodiment, the composition comprises a component selected from the group consisting of oils (optionally selected from vegetable oils, triglycerides, esters, and silicone oils; linear and cyclic oils; non-volatile and volatile oils), waxes (such as ozokerite, polyethylene wax, beeswax, candelilla or camauba wax), silicone elastomers, surfactants, preferably an emulsifying surfactant (either non-ionic, anionic, cationic or amphoteric), co-surfactants (such as linear fatty alcohols), thickeners, solidifiers, moisturizers (such as the polyols such as glycerin), organic filters, inorganic filters, dyes, preservatives, feedstocks, tightening agents, sequestering agents, perfumes, and any combination thereof. For example, useful additives or adjuvants are as cited in the Dictionary CTFA (International Cosmetic Ingredient Dictionary and Handbook, published by the Personal Care Product Council).

In one embodiment, the pharmacologic or therapeutic composition is a dermatologic composition.

In one embodiment, the process of preparation is a process of preparation of an autophagy activator and/or an iron-biding agent, or an autophagy activator having iron-binding properties, or an extract or a composition according to the invention.

In one embodiment of the process of preparation, the cell culture provided in step (a) is a liquid cell culture, preferably growing in the log phase. For example, step (a) comprises incubating the cells at a temperature of from about 30 to 55 °C, preferably overnight, and/or at a pH of about 2-3.

In one embodiment of the process of preparation, the cell culture provided in step (a) is a phototrophic or an autotrophic cell culture, preferably an autotrophic cell culture.

In one embodiment of the process of preparation, step (b) comprises separating the cultured cells from the liquid culture medium. For example, step (b) comprises centrifugation, filtration, e.g. ultra-filtration, sedimentation or flotation. Preferably, separation is effected by centrifugation of the cell culture suspension, e.g. at 4,000 rpm, 2.800 x g for 10 minutes.

In one embodiment of the process of preparation, step (b) comprises washing the cultured cells, preferably the separated cells, for example the cell pellet resulting from centrifugation. For example, the cells are washed with physiological saline or a buffered salt solution, e.g. once, twice, three times, or several times. For example, the process of washing the cells comprises separating the cells from the washing solution, e.g. by centrifugation, and re-suspending the resulting cell pellet in fresh culture medium, physiological saline, a buffered salt solution or water.

In one embodiment of the process of preparation, step (c) comprises disrupting the cultured cells by mechanical or chemical or thermal means, e.g. by freezing and thawing, or repeated freezing/thawing cycles; by means of osmolytic shock; by exposure to increased or lowered pressure, e.g. high-pressure homogenisation; by blending, e.g. using a bead mill, ball mill, homogenizer or extruder; by ultra-sonification; or by means of surfactants, detergents, chaotropic salts, or cytolytic enzymes. Preferably, disrupting the cells is effected by freezing and thawing of cells, more preferably three freezing/thawing cycles, or by ultra-sonification of cells, most preferably by treating cells with ultrasound for 30 seconds, followed by 10 seconds of cooling (six cycles) at 200 W.

In one embodiment of the process of preparation, the extract obtained in step (d) is a crude cell homogenate or lysate, a fraction of a cell homogenate or lysate, e.g. a supernatant, a soluble fraction, a particulate fraction, a pellet, a re-suspended pellet or an extract from a pellet. For example, the supernatant or soluble fraction comprises the cytosolic compartment of cells; the particulate fraction comprises outer membranes, intracellular membranes or organelles of cells. For example, the supernatant or soluble fraction is obtained by removing the particulate fraction by means of centrifugation, sedimentation or filtering off.

In one embodiment, the extract obtained in step (d) is a filtrate obtained from a cell homogenate or lysate.

In one embodiment, the process of preparation comprises a further step (e) of freezing, drying or freeze-drying of the extract obtained in step (d), e.g. drying by spray drying or using a belt dryer. For example, drying or freeze-drying comprises applying the extract to a support or carrier. Preferably, step (e) comprises freeze-drying of the extract obtained in step (d).

In one embodiment, the process of preparation comprises a further step (f) of reconstituting, e.g. re-suspending or resolving the dried or freeze-dried extract obtained in step (e).

In one embodiment, the process of preparation comprises further steps, for example, one or more steps of purifying the extract, or a step of adding to the extract an additive, e.g. a stabilizer or a preservative.

In one embodiment, e.g. of the compositions or uses, the skin is human skin. In another embodiment, the skin is non-human animal skin, for example of a coat-wearing animal, for example of a mammal.

In one embodiment, e.g. of the compositions or uses, the skin is the skin of an aged subject, e.g. at the age of about 30, 40, 50, 60, 70, 80, 90 years.

In one embodiment, e.g. of the compositions or uses, the skin is altered or aged skin.

In one embodiment, e.g. of the compositions or uses, the skin is chronologically adequate aged skin or premature aged skin.

In one embodiment, e.g. of the compositions or uses, the skin is photo-aged skin.

In one embodiment, e.g. of the compositions or uses, the signs of altered or aged skin are lines, wrinkles, pigmented spots, liver spots, solar lentigines, senile warts, or is parchment skin. In one embodiment, e.g. of the compositions or uses, the age related skin appearance is characterized by lines, wrinkles, pigmented spots, liver spots, solar lentigines, senile warts, or parchment skin.

In one embodiment, e.g. of the compositions or uses, improving age-related skin appearance comprises reversing, at least partly, the signs of aged skin.

In one embodiment, e.g. of the compositions or uses, the skin is facial skin or body skin. For example, the skin is skin of the mouth, neck, chest, back arms, and legs.

In one embodiment, e.g. of the compositions or uses, the skin is scalp skin comprising hair follicles. For example, the hair follicles have reduced or increased activity in respect of hair production. For example, the scalp skin additionally comprises scalp hair and/or body hair. For example, the scalp skin additionally comprises hair consisting of scalp hair or body hair.

In one embodiment, e.g. of the compositions or uses, the altered or aged skin is lysosome related altered or aged skin or is related or due to a decrease of lysosomal activity and/or autophagy in skin cells, preferably keratinocytes.

In one embodiment, e.g. of the compositions or uses, the altered or aged skin is related or due to enhanced cellular contents of iron, e.g. free iron, in skin cells, preferably keratinocytes.

In one embodiment of the uses, the subject is human. For example, the human is a male or a female. For example, the human is a baby, a child, an adult, e.g. at the age of about 20, 30, 40, or 50 years, or is an elderly person, e.g. at the age of about 50, 60, 70, 80 or 90 years. In another embodiment, the subject is a non-human animal, for example is a coat-wearing animal, for example a mammal.

In other embodiments, the uses are for the following:
(a) treatment, reduction, and/or prevention of fine lines or wrinkles,
(b) reduction of skin pore size,
(c) improvement in skin thickness, plumpness, and/or tautness;
(d) improvement in skin suppleness and/or softness;
(e) improvement in skin tone, radiance, and/or clarity;
(f) improvement in procollagen and/or collagen production;
(g) improvement in maintenance and remodelling of elastin;
(h) improvement in skin texture and/or promotion of re-texturization;
(i) improvement in skin barrier repair and/or function;
(j) improvement in appearance of skin contours;
(k) restoration of skin luster and/or brightness;
(l) replenishment of essential nutrients and/or constituents in the skin;
(m) improvement of skin appearance decreased by aging and/or menopause;
(n) improvement in skin moisturization and/or hydration;
(o) increase in and/or preventing loss of skin elasticity and/or resiliency;
(p) treatment, reduction, and/or prevention of skin sagging;
(q) treatment, reduction, and/or prevention of discoloration of skin;
(r) detoxification of the skin;
   and any combination thereof.

In a particular embodiment, the invention provides a method for improving the aesthetic appearance of skin of a human affected by aging resulting from reduced autophagy activity within skin cells of the human comprising topically applying thereto a therapeutically effective amount of the *Galdieria* extract in a cosmetically acceptable vehicle for a time sufficient to achieve an improvement in the appearance of said skin of said human.

In the following, the invention will be described in more detail with reference to the accompanying figures and by means of the examples which are for illustrative purposes only and do not intend to limit the scope of the claimed invention.

### Figures

- Fig. 1: shows the effect of *Galdieria* sp. whole cells and *Galdieria* extracts (ultrasonic homogenate, concentrated homogenate, re-suspended lyophilized homogenate) in different concentrations (0.5, 1 or 2%, by volume) on HaCaT cells, as measured in a neural red assay ([%]; control = untreated cells = 100%).
- Fig. 2: shows the effect of *Galdieria* sp. whole cells and *Galdieria* extracts in different concentrations on the amount of LC3B protein in HaCaT cells.
- Fig. 3: shows the effect of *Galdieria* extract (freezing/thawing lysate; 0.5, 1.0 or 2%, by volume) and of extracts (freezing/thawing lysates) from *Gloeocystis* sp. (0.5, 1.0 or 2%, by volume) or *Chlamydomonas reinhardtii* (1.0, 2.0 or 5%, by volume) on HaCaT cells, as measured in a neutral red assay and in terms of LC3B protein.
- Fig. 4: shows the effect of *Galdieria* sp. whole cells and *Galdieria* extracts in different concentrations on the intracellular ATP level of HaCaT cells.
- Fig. 5: shows the effect of *Galdieria* extract (freezing/thawing lysate; 0.1, 0.5, 1, or 2%, by volume) on HaCaT cells, as measured in a crystal violet assay.
- Fig. 6: shows the effect of spermidine (100 or 250 µM) on HaCaT cells in terms of LC3B protein and intracellular ATP level, and as measured in a neutral red assay, MTT assay or LDH assay.

### EXAMPLES

### EXAMPLE 1: Cultivation of Galdieria sp.

Stock cultures of *Galdieria sulphuraria* are obtainable from commercial culture collections, e.g. Culture Collection of Algae at the University of Göttingen, Germany (SAG) or Algal Collection University Federico II - Naples, Italy (ACUF). The used *Galdieria sulphuraria* culture was originally isolated from an Onsen in Japan.

The basic culture medium was one typically used for phototrophic algae cultivation:
4.5 g (NH₄)₂SO₄, 0.6 g MgSO₄ x 7H₂O, 0.6 g KH₂PO₄, 0.03 g CaCl₂ x 2H20, 0.01 g FeSO₄, 0.018 g ethylenediaminetetraacetic acid (EDTA) disodium dihydrate, 0.005 g H₃BO₃, 0.004 MnCl₂, 0.00068 g ZnSO₄ x 7H₂O, 0.00052 g Na₂MoO₄ x 2H₂O, 0.00008 g CuSO₄ x 5H₂O, 0.00008 g NaVO₃ x 4H₂O, 0.00064 g CoCl₂ x 6H₂O, per liter of distilled water.

An alternative culture medium is as suggested by Allen (1959).

The pH of the medium was brought to 2-3 by adding 1 N H₂SO₄ and/or by adjustment of CO₂.

*Galdieria* was grown under heterotrophic conditions. For heterotrophic cultivation, the basic culture medium was modified by adding up to 10% of an organic carbon source, e.g. glycerol, glucose, vinasse, molasses, galactose, or fructose.

The cultivation temperature was between 30-55°C.

### EXAMPLE 2: Preparation of Galdieria extract

*Galdieria* sp. biomass was harvested from liquid culture medium by centrifugation (4,000 rpm, 2,800 x g, 10 minutes), and was washed with a buffered salt solution.

Cell pellets were re-suspended in physiological saline (0.9% NaCl, aqueous solution), and the cells were lysed by three freezing/thawing cycles. As a result, *Galdieria* extract in terms of a lysate was obtained.

Alternatively, the re-suspended cells were treated by ultrasound for 30 seconds, followed by 10 seconds of cooling (six cycles) at 200 W. As a result, *Galdieria* extract in terms of a homogenate or ultrasonic homogenate was obtained.

For some applications, a threefold concentrated extract was prepared from a concentrated cell culture suspension (by means of sedimentation or ultrafiltration) using high-pressure homogenization (> 800 bar). As a result, Galdieria extract in terms of a concentrated homogenate was obtained.

Furthermore, soluble and particulates extracts were prepared from homogenates by centrifugation and separation of the (soluble) supernatant from the (particulate) pellet. The autophagy activity was found both in the soluble and particulate fraction.

For storage purposes, lysates or homogenates were lyophilized. When needed, a lyophilisate was re-suspended in physiological saline, and the resuspension was used as *Galdieria* extract. Such reconstituted lyophilisates were similarly concentrated as the lysates or homogenates from which they originated.

### EXAMPLE 3: Chlorophyta extracts

For comparison with *Galdieria* extract, two species of green algae (Chlorophyta), namely *Gloeocystis* sp. and *Chlamydomonas reinhardtii* (obtained from Culture Collection of Algae and Protozoa, CCAP, UK) were cultured and extracts thereof were prepared.

The culture medium was as follows (Krienitz and Wirth, 2006):
KNO₃ 200 mg/l, K₂HPO₃ 40 mg/l, MgSO₄ · 7H₂O 30 mg/l, Ca(NO₃)₂ 30 mg/l, NaHCO₃ 168 mg/l; Fe/EDTA solution: FeSO₄ · 7H₂O 3.5 mg/l; EDTA (Titriplex III) 4.5 mg/l; vitamins: biotin 0.33 mg/l, vitamin B12 0.05 mg/l, thiamin 0.05 mg/l; trace elements: MnCl₂ · 4H₂O 4.95 mg/l, CoSO₄ · 7H₂O 0.14 mg/l, CuSO₄ · 5H₂O 0.025 mg/l, ZnSO₄ · 7H₂O 0.36 mg/l, H₃BO₃ 0.155 mg/l, (NH₄)₆Mo₇O₂₄ · 4H₂O 0.09 mg/l, NiSO₄ · 6H₂O 0.1315 mg/l, NH₄VO₃ 0.075 mg/l.

Alternative culture media are TAP medium (Gorman and Levine, 1965) and Bold's Basal medium (Bischoff and Bold, 1963).

The light regime for cultivating the green algae was adjusted and controlled as appropriate.

Extracts from *Gloeocystis* sp. or *Chlamydomonas reinhardtii,* i.e. Chlorophyta extracts, were prepared similarly to the *Galdieria* extract by three freezing/thawing cycles (see Example 2).

### EXAMPLE 4: Skin cell in vitro assay

Human keratinocytes (HaCaT) were grown in Dulbeco's Modified Eagle Medium (DMEM), supplemented with 5% FCS. Cells were harvested in the stationary phase. Prior to trypsination, the cells were pre-treated with EDTA solution.

A cell suspension was prepared in DMEM, the cells were seeded in flat bottom microtiter plates (1.8 · 10⁴ cells /well), and the cell cultures were incubated at 37°C, 5% CO₂. Twenty-four hours after seeding, *Galdieria* or Chlorophyta extract, or spermidine was added to the cell cultures. Untreated cells served as controls.

After a further 48 hours of incubation, the culture supernatant was removed and the microtiter plate was gently tapped on a paper towel. The microtiter plate was washed twice with 200 µl/well of phosphate buffered saline (PBS).

At this stage, further treatments as described in Examples 5, 6, 8, 11, and 14 to 17 were carried out.

### EXAMPLE 5: DAPI cell staining

Cell staining with DAPI (4',6-diamidino-2-phenylindol) is a measure of cell count and served for cell count correction.

DAPI is a fluorescent dye that is used in fluorescence microscopy to label DNA. The compound accumulates preferentially to AT-rich regions in the minor groove of DNA. When excited with ultraviolet light, DAPI fluoresces with blue to cyan color. In connection with double-stranded DNA, the absorption maximum is at a wavelength of 358 nm, the emission maximum at 461 nm.

HaCaT cells were grown and treated as described in Example 4. A methanol solution with 0.0005% DAPI was applied to the cell layer for 15 minutes at 37°C. Subsequently, the cells were washed with double distilled water and the microtiter plate was allowed to dry for 30 minutes at 37°C.

The fluorescence was measured at 355 nm (excitation) and 600 nm (emission) in a fluorescence reader (Fluoroscan Ascent FL; Labsystems).

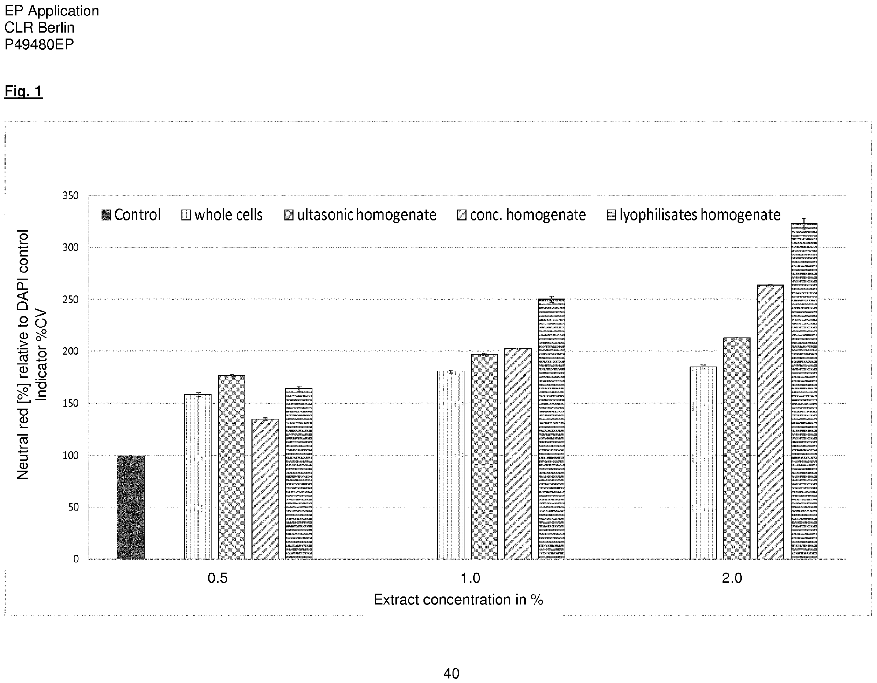

All data in Figs. 1 to 6 and in Table 1 further below are DAPI corrected.

### EXAMPLE 6: Neutral red assay

Lysosomal activity in keratinocytes was measured using a neutral red assay described by Al-Qenaei et al. (2014) for determining lysosomal integrity.

Neutral red is a cationic dye, which diffuses into the cell and binds to the lysosomal matrix. Inside the lysosome, neutral red changes the charge and color due to the acidic pH. The dye accumulates following the principle of ion trap. Bound dye can be re-dissolved and the colored solution is measured at 570 nm in a microplate reader. The OD at 570 nm corresponds to lysosomal activity.

HaCaT cells grown and treated as described in Example 4 were stained by adding to each well 0.005% of a neutral red solution in DMEM. After 4 hours at 37°C, 5% CO₂, the microtiter plate was carefully washed tree times with 200 µl/well PBS and the stain was redissolved with 50% acidic ethanol in double-distilled H₂O. Absorbance was read in a microplate reader at 570 nm.

### EXAMPLE 7: Effect of Galdieria extract on lysosomal activity

As shown in Fig. 1, *Galdieria* sp. (whole cells) and *Galdieria* extracts induced an increase in neutral red staining of HaCaT cells.

The effect was less pronounced with whole cells than with cell homogenates. Notably, the effect was maintained with re-suspended homogenates after lyophilization, and this effect was dose-dependent.

Theses findings indicate that *Galdieria* extract stimulates lysosomal activity and thus autophagy in keratinocytes.

### EXAMPLE 8: LC3B assay

Autophagy in keratinocytes was tested in an ELISA assay based on the mammalian autophagy protein LC3B, a marker of cellular autophagosomes. Organelles to be digested are embedded by membranes which form the autophagosome. This LC3B containing autophagosome fuses with the lysosome forming the autolysosome to degrade enclosed components. After fusion of autophagosome and lysosome the membrane protein LC3B is no longer detectable. Therefore, fusion must be prevented by the inhibitor chloroquine which serves as an interrupter of the autophagy flux and consequently as a collector of autophagosome containing LC3B protein.

HaCaT cells were grown and treated as described in Example 4. Simultaneously with *Galdieria* or Chlorophyta extract, or spermidine, 2 µM chloroquine diphosphate salt (Sigma) was added to the cell cultures. As described in Example 4, the cell cultures were incubated at 37°C, 5% CO₂, for 48 hours, the culture supernatant was removed, and the microtiter plate was washed with PBS. Then, a fixation solution (4% formaldehyde) was added to the cells (200 µl/well) for 15 minutes at room temperature.

After repeated washing with PBS, cells were permeabilized by adding 150 µl/well methanol for 10 minutes at -20°C.

After repeated washing with PBS, protein was blocked by adding 300 µl/well blocking solution (5% goat serum, 0.3% Triton X-100 in PBS) for 60 minutes at room temperature.

Then, an antibody solution (LC3B (D11) XP® Rabbit mAb; NEB) was added (100µl/well) and the microtiter plate was incubated at 4°C for 18 hours.

For labeling with the secondary antibody, the microtiter plate was washed again and 100 µl/well of a secondary antibody solution (Anti-Rabbit IgG (H+L), F(ab')2 Fragment (Alexa®488)) was added.

After repeated washing, fluorescence was measured at 485 nm (excitation) and 535 nm (emission) in a fluorescence reader (Spectramax Paradigm).

### EXAMPLE 9: Effect of Galdieria extract on autophagy

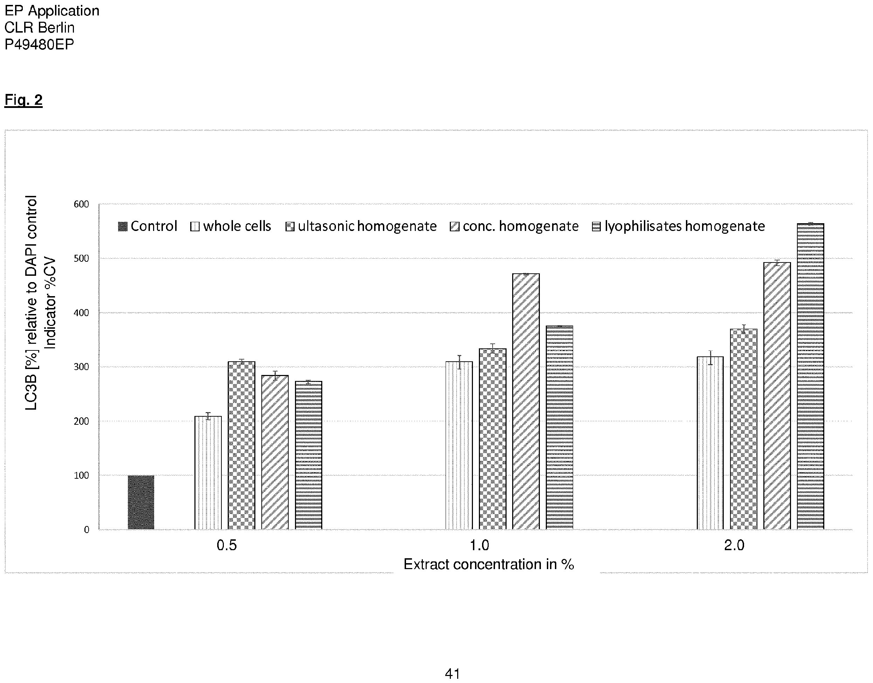

As shown in Fig. 2, *Galdieria* sp. (whole cells) and *Galdieria* extracts induced an increase in LC3B protein in HaCaT cells.

The effect was less pronounced with whole cells than with cell homogenates. The effect was particularly pronounced with homogenate concentrates at 1% and 2%, and most pronounced with re-suspended homogenates after lyophilization at 2%.

These findings indicate that *Galdieria* extract stimulates autophagy in keratinocytes.

### EXAMPLE 10: Comparison of Galdieria and Chlorophyta extract

As shown Figs. 1 and 2, *Galdiera* extract was capable of increasing lysosomal activity and LC3B protein in keratinocytes.

Without wishing to be bound to this theory, we postulated that the observed effects may be due to glycosylation, which, however, would not match with the evolutionary distance of *Gadieria* to mammalian cells. For evaluation of this hypothesis, we tested Chlorophyta, i.e. predecessors of land plants, which are a closer to mammalian cells in terms of glycosylation.

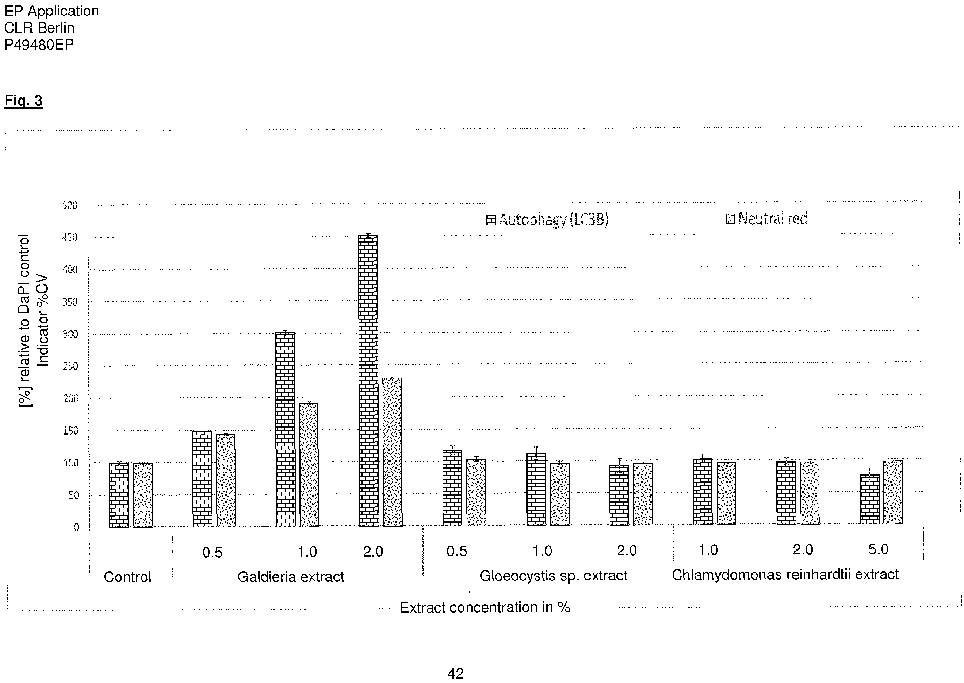

As shown in Table 1 below and Fig. 3, Chlorophyta extracts of up to 5% had virtually no effect on lysosomal activity and LC3B protein in HaCaT cells.

**Table 1: Neutral red activity of Galdieria and Chlorophyta extracts relative to an untreated control (normalized to DAPI)**

| Concentration of extract [%, by vol.]* | *Galdieria.* | Chlorophyta |
|---|---|---|
| 0.50 | 150.2 | 105.1 |
| 1.00 | 194.0 | 103.0 |
| 2.00 | 242.1 | 100.8 |

These findings indicate that *Galdieria* extract is remarkable in its capability of stimulating lysosomal activity and autophagy in keratinocytes.

### EXAMPLE 11: ATP assay

Detoxification of the cell involves catablolic activity and consumption of ATP. A decrease of the intracellular ATP level was found to be associated with an aged or senescent cell (Brunk and Terman, 2002).

The intracellular ATP level of keratinocytes in response to *Galdieria* extract was measured using ATPLite-M, a chemiluminescent detection assay (Luminescent Adenosine Triphosphate Detection Assay, Packard, Cat. No. 6016541). The ATP Lite-M monitoring system is based on fire-fly luciferase and the production of light caused by enzyme catalyzed reaction of ATP and D-luciferin.

HaCaT cells were grown and treated as described in Example 4. After cell lysis, the substrate solution was added. Then, the plate was adapted to darkness for 10 minutes. The luminescence was measured in a luminescence reader (Fluoroscan Ascent FL; Labsystems).

### EXAMPLE 12: Effect of Galdieria extract on intracellular ATP

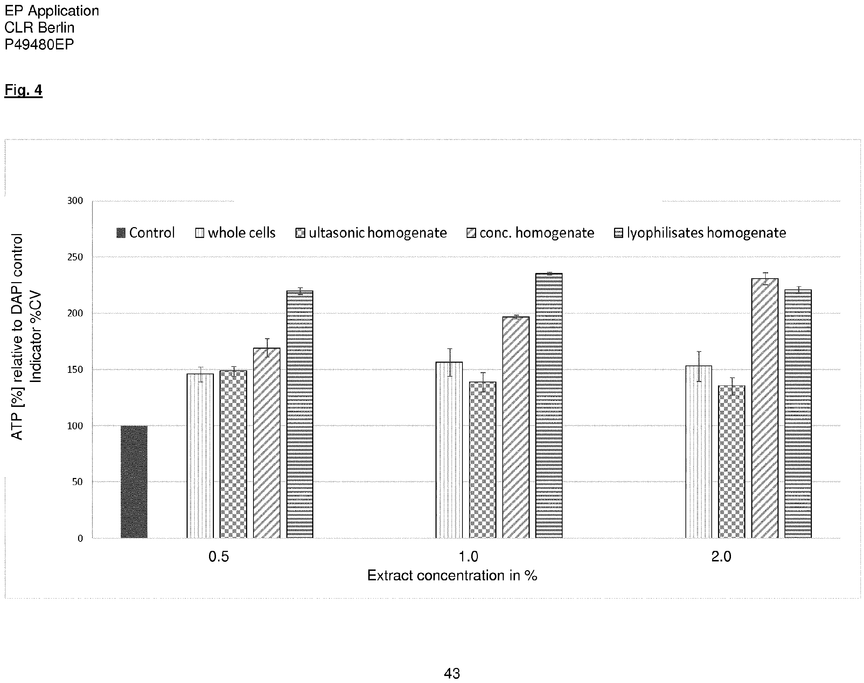

As shown in Fig. 4, *Galdieria* sp. (whole cells) and *Galdieria* extract increased the intracellular ATP level of HaCaT cells. The effect was maintained in re-suspended homogenates after lyophilization.

These findings confirm that *Galdieria* extract stimulates lysosomal turnover in keratinocytes.

### EXAMPLE 13: Iron chelating properties of Galdieria extract

Surprisingly, *Galdieria* extract was shown to have the capability of slowing down the Fenton reaction by 32% in an experimental set up with Fe²⁺ and H₂O₂. Due to the low result of the antioxidative power (AP = 0) it can be concluded that this effect was not based on a direct antioxidative scavenging of the H₂O₂. Therefore, the decelerated Fenton reaction is most probably related to a reduction of the availability of the catalyst.

This finding indicates that *Galdieria* extract may contain a component that binds to and thus reduces free iron.

### EXAMPLE 14: Crystal violet assay

The dye hexamethylpararosaniline chloride (crystal violet) unspecifically binds to proteins. When re-dissolved, the coloured solution is measurable in an ELISA reader. In this way, changes in the amount of cellular protein can be visualized by changes in the OD at 570 nm. As cell profliferation involves an increase in protein, the crystal violet assay indicates inhibitory effects on cell proliferation or cytotoxic effects.

HaCaT cells were grown and treated as described in Example 4. After the PBS washing step, the microtiter plate was allowed to dry at 37°C for 30 minutes. Then, the cells were stained with 75 µl/well of crystal violet solution (5%) at room temperature for 5 minutes. The dye solution was removed by tapping and excess dye was removed by washing with double-destilled water. Again, the plate was allowed to dry at 37°C for 30 minutes. Bound dye was re-dissolved by adding 25 µl/well of phosphoric acid (0.2 M) and 75 µl/well of ethanol. After shaking for 5 minutes, the OD was measured using a microplate reader (Spectramax Paradigm) at 570 nm, reference 630 nm.

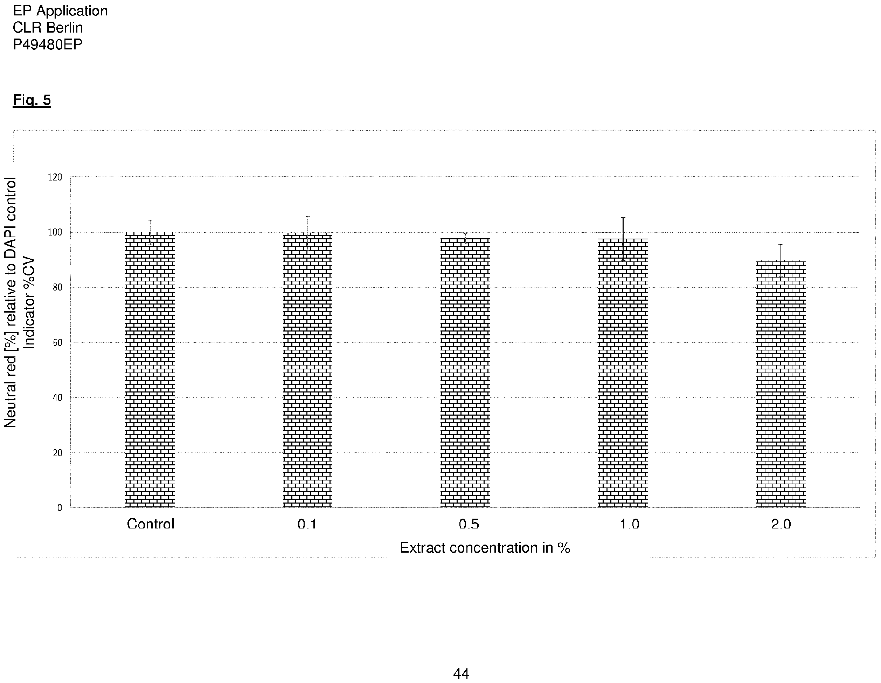

As shown in Fig. 5, *Galdieria* extract exerted no inhibitory or cytotoxic effects on HaCaT cells.

### EXAMPLE 15: MTT assay

The MTT assay was used for determining HaCaT cell metabolic activity and viability in the presence of spermidine.

In the mitochondria where dehydrogenases are localized the oxidative metabolic process of the cell takes place (citric acid cycle). This process aims at converting oxidation energy into energy-rich ATP (adenosine triphosphate) by oxidative phosphorylation. Since this requires involvement of dehydrogenases, there is a direct relation between increased dehydrogenase activity, enhanced metabolism and increased cellular energy potential.

The yellowish tetrazolium salt 3-(4,5-dimethylthiazol-2-yl)-2,5-diphenyltetrazoliumbromide (MTT) is reduced to dark-blue formazan through ring cleavage by mitochondrial succinate dehydrogenases. Intensity of this colour change is measurable at 570 nm.

HaCaT cells were grown and treated as described in Example 4. Then, 10µl/well of a MTT-solution (5mg/ml PBS) were added to the cells for a further 4 hours. Cell supernatants were removed and cell bound formazan was dissolved by adding 100 µl/well of a stop-solution containing 99.4 ml DMSO (dimethyl sulfoxide, Sigma), 10 g SDS (sodium dodecyl-sulphate, Sigma) and 0.6 ml acetic acid. The absorbance was read in a microplate reader (Spectramax Paradigm) at 570 nm, reference 630 nm.

### EXAMPLE 16: LDH assay

The LDH assay was used for determining HaCaT cell integrity and viability in the presence of spermidine.

Lactate dehydrogenase (LDH) is a stable cytoplasmic enzyme which is rapidly released into the cell culture medium when the plasma membrane is damaged. LDH activity is determined using a tetrazolium salt (INT) which is enzymatically reduced to formazan. The formazan dye is water-soluble and has a broad absorption maximum at approximately 500 nm.

HaCaT cells were grown and treated similar to the description in Example 4. After 48 hours of incubation with spermidine, the cell supernatant collected. For that purpose, the microtiter plate was centrifuged at 250 x g for 10 minutes. Cell-free supernatant (100 µl/well) was transferred to a fresh, optically clear flat bottom microtiter plate. Then, 100 µl of reaction mixture was added to the 100 µl supernatant in each well. The plate was incubated in the dark for 30 minutes at room temperature. The absorbance was measured at 490 nm, reference 600 nm in a microplate reader (Spectramax Paradigm).

### EXAMPLE 17: Effect of spermidine on lysosomal activity and autophagy

Spermidine is reported to induce autophagy, and spermidine is expressed in *Galieria* sp. Thus, the question arose whether the effects observed with *Galdieria* extract were related to the presence of algal spermidine.

HaCaT cells were grown (1.3 · 10⁴ cells/well) and treated with spermidine (100 or 250 µM) as described in Example 4.

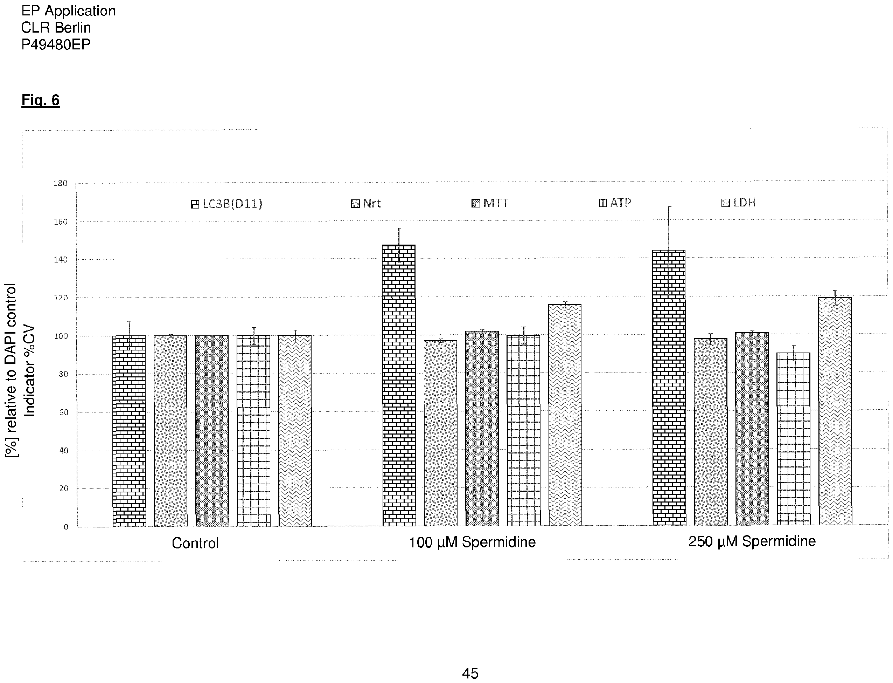

As shown in Fig 6, spermidine induced an increase in LC3B protein in HaCaT cells, while lysosomal activity (measured using the neutral red assay) remained unaffected. Metabolic activity of HaCaT cells (measured using the MTT assay) was not affected either, and cell integrity (LDH release) was only slightly impaired.

Notably, however, the increase in LC3B protein amounted only to about 1.5-fold, and there was no difference between 100 µM and 250 µM spermidine. In contrast, *Galdieria* extract induced a dose-dependent increase in LC3B protein by up to about 4.5-fold (see, e.g., Fig. 3). This difference between the findings with pure spermidine and *Galdieria* extract is even more remarkable when considering that 100 µM or 250 µM spermidine are concentrations which are quite high and certainly not to be expected to occur endogenously in the extracts.

Therefore, it is concluded that *Galdieria* extract contains autophagy activities, which are not due to the presence of spermidine.

### EXAMPLE 18: Cosmetic compositions

In the following, various cosmetic compositions containing *Galdieria* extract are presented by way of example. All ingredients (given in %, by weight) are in accordance with the International Nomenclature of Cosmetic Ingredients (INCI).

### Example 18.1: Skin gel ("moisturizing after sun cooling gel")

| | |
|---|---|
| Polysorbate 20 | 2.5 |
| Alcohol | 15.0 |
| Bisabolol | 0.1 |
| Panthenol | 2.0 |
| Glycerin | 4.0 |
| Sodium carbomer | 0.5 |
| *Galdieria* extract | 0.3 |
| Aqua dem. | ad 100 |

### Example 18.2: Serum or liquid ("face cleanser mask")

| | |
|---|---|
| Hydrolized corn starch (and) Beta vulgaris root extract (Daymoist) | 3.0 |
| Polyacrylate crosspolymer-11 (Aristoflex Velvet) | 2.0 |
| Decyl glucoside | 2.5 |
| Coco-glucoside (and) glyceryl oleate (Lamesoft PO65) | 3.0 |
| Sodium benzoate | 0.4 |
| Polylactic acid | 2.0 |
| Sodium benzoate | 0.4 |
| Potassium sorbat | 0.3 |
| Citric acid | 0.05 |
| *Galdieria* extract | 0.3 |
| Aqua dem. | ad 100 |

### Example 18.3: Emulsified gel ("face defend cream gel")

| | |
|---|---|
| Arachidyl alcohol (and) behenyl alcohol (and) | |
| Arachidyl glucoside (Montanov 202) | 3.0 |
| Isopropyl palmitate | 5.0 |
| Caprylic/capric triglyceride | 10.0 |
| Polyacrylamide, laureth-7, C₁₃₋₁₄ Isoparaffin (Sepigel 305) | 2.0 |
| Phenoxyethanol | 0.9 |
| Ethylhexylglycerin | 0.1 |
| *Galdieria* extract | 0.3 |
| Aqua dem. | ad 100 |

### Example 18.4: Non-ionic emulsion ("body lotion")

| | |
|---|---|
| Cetearyl glucoside and cetearyl alcohol (Montanov 68) | 5.0 |
| Isostearyl isostearate | 5.0 |
| Caprylic/capric triglyceride | 10.0 |
| Butyrospermum parkii (shea) butter | 1.5 |
| Xantan gum | 0.5 |
| Phenoxyethanol | 0.9 |
| Ethylhexylglycerin | 0.1 |
| *Galdieria* extract | 0.3 |
| Aqua dem. | ad 100 |

### Example 18.5: Cationic emulsion ("hand care cream")

| | |
|---|---|
| Distearyldimonium Chloride | 5.0 |
| Caprylic/Capric Triglyceride | 5.0 |
| Squalane | 10.0 |
| Dimethicone | 0.5 |
| Hydroxyethylcellulose | 0.2 |
| Glycerin | 4.0 |
| Phenoxyethanol | 0.9 |
| Ethylhexylglycerin | 0.1 |
| *Galdieria* extract | 0.3 |
| Aqua dem. | ad 100 |

### Example 18.6: Anionic emulsion ("protecting foot care cream")

| | |
|---|---|
| Glyceryl stearate SE | 5.0 |
| Stearyl alcohol | 1.0 |
| Stearic acid | 2.0 |
| Simmondsia chinensis (Jojoba) Seed Oil | 5.0 |
| Caprylic/capric triglyceride | 10.0 |
| Isononyl isononanoate | 5.0 |
| Glycerin | 3.0 |
| Ethylhexylglycerin | 0.1 |
| *Galdieria* extract | 0.3 |
| Aqua dem. | ad 100 |

### Example 18.7: Emulsion w/o ("nappy rash cream")

| | |
|---|---|
| Cetyl PEG/PPG-10/1 dimethicone | 2.0 |
| Polyglyceryl-4 isostearate | 1.0 |
| Stearoxy dimethicone | 3.0 |
| Squalene | 10.0 |
| Simmondsia chinensis (Jojoba) seed oil | 2.0 |
| Petrolatum | 10.0 |
| Hydrogenated castor oil | 1.5 |
| Microcrystalline wax | 2.0 |
| Zinc oxide | 10.0 |
| Sodium chloride | 0.5 |
| Glycerin | 3.0 |
| Panthenol | 0.5 |
| Sodium benzoate | 0.3 |
| Phenethyl alcohol | 0.5 |
| *Galdieria* extract | 0.3 |
| Aqua dem. | ad 100 |

### Example 18.8: Emulsion w/o ("anti-aging foundation")

| | |
|---|---|
| Cetyl PEG/PPG-10/1 dimethicone | 3.0 |
| PPG-3 myristyl ether | 5.0 |
| Dicaprylyl carbonate | 10.0 |
| C₁₂₋₁₅ alkyl benzate | 5.0 |
| Talc | 1.5 |
| Iron oxide | 1.9 |
| Titanium dioxide | 5.0 |
| Sodium chloride | 0.8 |
| Glycerin | 3.0 |
| Sodium benzoate | 0.3 |
| Phenethyl alcohol | 0.5 |
| *Galdieria* extract | 0.3 |
| Aqua dem. | ad 100 |

### Example 18.9: Emulsion w/o ("anti-aging cream")

| | |
|---|---|
| Cetearyl glucoside and cetearyl alcohol (Montanov 68) | 5.0 |
| Cetearyl alcohol | 0.5 |
| Caprylic/capric triglyceride | 8.0 |
| Isononyl isononanoate | 8.0 |
| Butylene glycol | 4.0 |
| Sodium carbomer | 0.2 |
| Phenoxyethanol | 0.9 |
| Ethylhexylglycerin | 0.1 |
| *Galdieria* extract | 0.3 |
| Aqua dem. | ad 100 |

### Example 18.10: Pickering emulsion ("mat lipstick red")

| | |
|---|---|
| Butylene glycol | 3.0 |
| Phenoxyethanol | 0.4 |
| Polyurethane-62 (and) trideceth-6 (Flex-6 Polymer) | 1.0 |
| Diisostearyl dimer dilinoleate | 35.0 |
| Copernicia cerifera (Carnuba) wax | 1.0 |
| Euphorbia cerifera (Candelilla) wax | 2.9 |
| Ozokerite | 11.7 |
| Microcrystalline wax | 3.6 |
| Paraffin | 2.9 |
| Ricinus communis (Castor) seed oil (and) CI 15850 | 3.8 |
| Ricinus communis (Castor) seed oil (and) CI 77891850 | 12.0 |
| Mica (and) titanium oxide | 6.2 |
| *Galdieria* extract | 0.3 |
| Aqua dem. | ad 100 |

### Example 18.11: Pressed powder ("skin care powder SPF")

| | |
|---|---|
| Talc | 46.2 |
| Mica | 12.9 |
| Zinc oxide (Tego Sun Z 500) | 5.7 |
| Titanium dioxide (Sun Chroma) | 6.0 |
| Nylon-12 (Orgasol 2002) | 6.0 |
| Magnesium stearate | 1.0 |
| Mica (and) iron oxides (Prestige Soft Copper) | 0.25 |
| Mica (and) iron oxides (Prestige Soft Silver) | 0.25 |
| Mica (and) titanium dioxide (and) iron oxides (Prestige Soft Gold) | 7.4 |
| Methylparaben | 0.3 |
| Glycerin | 1.0 |
| Cyclopentasiloxane/dimethiconol (Dow Corning 1501) | 6.0 |
| Caprylic/capric triglyceride | 3.0 |
| Glyceryl cocoate/citrate/lactate (Imwitor 380) | 1.0 |
| *Galdieria* extract | 0.3 |

### Example 18.12: Powder aerosol ("skin protecting body spray")

| | |
|---|---|
| Isopropylmyristate | 5 |
| Propane, Butane, Isobutan (Propellant) | 94.7 |
| *Galdieria* extract | 0.3 |

### Example 18.13: Non-aerosol spray ("sun protection spray SPF 30")

| | |
|---|---|
| Glycerin | 3.0 |
| Panthenol | 0.5 |
| Acrylates/C₁₀₋₃₀ alkyl acrylate crosspolymer | 0.1 |
| Microcrystalline cellulose | 0.8 |
| Phenylbenzimidazole sulfonic acid | 1.0 |
| Aminomethyl propanol | 0.38 |
| Xanthan gum | 0.05 |
| Sodium stearoyl glutamate | 0.4 |
| Glycerin | 0.8 |
| Cetyl alcohol | 0.8 |
| Octyl salicylate | 5.0 |
| Dicaprylyl ether | 2.0 |
| Homosalate | 6.0 |
| C₁₂₋₁₅ Alky benzoate | 3.0 |
| Avobenzone | 3.0 |
| Octocrylene | 5.0 |
| Dimethicone | 2.0 |
| Cyclomethicone | 2.0 |
| Phenoxyethanol | 0.9 |
| Benzylalkohol | 0.5 |
| VP/Hexadecene copolymer | 0.5 |
| Tetrasodium EDTA | 0.05 |
| *Galdieria* extract | 0.3 |
| Aqua dem. | ad 100 |

### References

Akinduro, O., Sully, K., Patel, A., Robinson, D.J., Chikh, A., McPhail, G., Braun, K.M., Philpott, M.P., Harwood, C.A., Byrne, C., O'Shaughnessy, R.F.L., Bergamaschi, D., 2016. Constitutive Autophagy and Nucleophagy during Epidermal Differentiation. J. Invest. Dermatol. 136, 1460-1470. doi:10.1016/j.jid.2016.03.016
Allen, M.B., 1959. Studies with Cyanidium caldarium, an anomalously pigmented chlorophyte. Arch. Microbiol. 32, 270-277.
Al-Qenaei, A., Yiakouvaki, A., Reelfs, O., Santambrogio, P., Levi, S., Hall, N.D., Tyrrell, R.M., ourzand, C., 2014. Role of intracellular labile iron, ferritin, and antioxidant defence in resistance of chronically adapted Jurkat T cells to hydrogen peroxide. Free Radic. Biol. Med. 68, 87-100. doi:10.1016/j.freeradbiomed.2013.12.006
Baird, S.K., Kurz, T., Brunk, U.T., 2006. Metallothionein protects against oxidative stress-induced lysosomal destabilization. Biochem. J. 394, 275-283. doi:10.1042/BJ20051143
Bimonte, M., De Lucia, A., Carola, A., Tito, A., Guoni, S., Langellotti, L., Fogliano, V., Colucci, G., Apone, F., 2016. Galdieria sulphuraria relieves oily and seborrheic skin by inhibiting the 5-α reductase expression in skin cells and reducing sebum production in vivo. Trichol. Cosmetol. Open. J. 1, 11-18.
Bischoff, H.W. and Bold, H.C., 1963: Phycological studies. IV. Some soil algae from Enchanted Rock and related algal species. - University of Texas Publications 6318: 1-95)
Brunk, U.T., Terman, A., 2002. The mitochondrial-lysosomal axis theory of aging: accumulation of damaged mitochondria as a result of imperfect autophagocytosis. Eur. J. Biochem. FEBS 269, 1996-2002.
Bulterijs, S., Hull, R.S., Björk, V.C.E., Roy, A.G., 2015. It is time to classify biological aging as a disease. Front. Genet. 6. doi:10.3389/fgene.2015.00205
Cuervo, A.M., Dice, J.F., 2000. When lysosomes get old. Exp. Gerontol. 35, 119-131.
Cuervo, A.M., Macian, F., 2012. Autophagy, nutrition and immunology. Mol. Aspects Med. 33, 2-13. doi:10.1016/j.mam.2011.09.001
De Domenico, I., Ward, D.M., Kaplan, J., 2009. Specific iron chelators determine the route of ferritin degradation. Blood 114, 4546-4551. doi:10.1182/blood-2009-05-224188
Dixon, S.J., Lemberg, K.M., Lamprecht, M.R., Skouta, R., Zaitsev, E.M., Gleason, C.E., Patel, D.N., Bauer, A.J., Cantley, A.M., Yang, W.S., Morrison, B., Stockwell, B.R., 2012. Ferroptosis: An Iron-Dependent Form of Non-Apoptotic Cell Death. Cell 149, 1060-1072. doi:10.1016/j.cell.2012.03.042
Elias, P., Ahn, S., Brown, B., Crumrine, D., Feingold, K.R., 2002. Origin of the epidermal calcium gradient: regulation by barrier status and role of active vs passive mechanisms. J. Invest. Dermatol. 119, 1269-1274. doi:10.1046/j.1523-1747.2002.19622.x
Fransen, M., Nordgren, M., Wang, B., Apanasets, O., 2012. Role of peroxisomes in ROS/RNS-metabolism: implications for human disease. Biochim. Biophys. Acta 1822, 1363-1373. doi:10.1016/j.bbadis.2011.12.001
Gorman, D.S., and R.P. Levine, 1965. Proc. Natl. Acad. Sci. USA 54, 1665-1669.
Gross, W., Küver, J., Tischendorf, G., Bouchaala, N., Büsch, W., 1998. Cryptoendolithic growth of the red alga Galdieria sulphuraria in volcanic areas. Eur. J. Phycol. 33, 25-31. doi:null
Hou, W., Xie, Y., Song, X., Sun, X., Lotze, M.T., Zeh, H.J., Kang, R., Tang, D., 2016. Autophagy promotes ferroptosis by degradation of ferritin. Autophagy 1-4. doi:10.1080/15548627.2016.1187366
Jain, K., Krause, K., Grewe, F., Nelson, G.F., Weber, A.P.M., Christensen, A.C., Mower, J.P., 2014. Extreme Features of the Galdieria sulphuraria Organellar Genomes: A Consequence of Polyextremophily? Genome Biol. Evol. 7, 367-380. doi:10.1093/gbe/evu290
Krienitz L; Wirth M, 2006. The high content of polyunsaturated fatty acids in Nannochloropsis limnetica (Eustigmatophyceae) and its implication for food web interactions, freshwater aquaculture and biotechnology. Limnologica, 36: 204-210.
Kurz, T., Eaton, J.W., Brunk, U.T., 2011. The role of lysosomes in iron metabolism and recycling. Int. J. Biochem. Cell Biol. 43, 1686-1697. doi:10.1016/j.biocel.2011.08.016
Kurz, T., Terman, A., Gustafsson, B., Brunk, U.T., 2008. Lysosomes in iron metabolism, ageing and apoptosis. Histochem. Cell Biol. 129, 389-406. doi:10.1007/s00418-008-0394-y
López-Otín, C., Blasco, M.A., Partridge, L., Serrano, M., Kroemer, G., 2013. The Hallmarks of Aging. Cell 153, 1194-1217. doi:10.1016/j.cell.2013.05.039
Nagashima, H., 1994. Natural products of the Cyanidiophyceae. In: Seckbach, J. (ed.) Evolutionlary pathways and enigmatic algae: Cyanidium caldarium (Rhodophta) and related cells. Vol. 91, chapter 16, p. 201-2014.
Pernodet, N., Dong, K., Pelle, E., 2016. Autophagy in human skin fibroblasts: Comparison between young and aged cells and evaluation of its cellular rhythm and response to Ultraviolet A radiation. J. Cosmet. Sci. 67, 13-20.
Pietrocola, F., Lachkar, S., Enot, D.P., Niso-Santano, M., Bravo-San Pedro, J.M., Sica, V., Izzo, V., Maiuri, M.C., Madeo, F., Mariño, G., Kroemer, G., 2015. Spermidine induces autophagy by inhibiting the acetyltransferase EP300. Cell Death Differ. 22, 509-516. doi:10.1038/cdd.2014.215
Raymond, A.-A., Gonzalez de Peredo, A., Stella, A., Ishida-Yamamoto, A., Bouyssie, D., Serre, G., Monsarrat, B., Simon, M., 2008. Lamellar bodies of human epidermis: proteomics characterization by high throughput mass spectrometry and possible involvement of CLIP-170 in their trafficking/secretion. Mol. Cell. Proteomics MCP 7, 2151-2175. doi:10.1074/mcp.M700334-MCP200
Rinnerthaler, M., Bischof, J., Streubel, M.K., Trost, A., Richter, K., 2015. Oxidative Stress in Aging Human Skin. Biomolecules 5, 545-589. doi:10.3390/biom5020545
Rubinsztein, D.C., Mariño, G., Kroemer, G., 2011. Autophagy and aging. Cell 146, 682-695. doi:10.1016/j.cell.2011.07.030
Rupar, C.A., Albo, S., Whitehall, J.D., 1992. Rat liver lysosome membranes are enriched in alpha-tocopherol. Biochem. Cell Biol. Biochim. Biol. Cell. 70, 486-488.
Settembre, C., Fraldi, A., Medina, D.L., Ballabio, A., 2013. Signals from the lysosome: a control centre for cellular clearance and energy metabolism. Nat. Rev. Mol. Cell Biol. 14, 283-296. doi:10.1038/nrm3565
Xie, Y., Hou, W., Song, X., Yu, Y., Huang, J., Sun, X., Kang, R., Tang, D., 2016. Ferroptosis: process and function. Cell Death Differ. 23, 369-379. doi:10.1038/cdd.2015.158
Xu, H., Ren, D., 2015. Lysosomal physiology. Annu. Rev. Physiol. 77, 57-80. doi:10.1146/annurev-physiol-021014-071649

## Claims

1. An extract from *Galdieria sulphuraria,* comprising an autophagy activator and/or an iron binding agent, or an autophagy activator having iron binding properties, wherein the extract contains cell debris.

2. The extract according to claim 1, which is an aqueous cell homogenate or lysate, or a particulate fraction thereof, or a filtrate thereof.

3. The extract according to claim 1 or 2, which underwent drying, preferably freeze-drying.

4. A cosmetic composition comprising an extract according to any of claims 1 to 3.

5. The cosmetic composition according to claim 4, which is adapted to topical application to the skin of a subject.

6. A non-therapeutic use of an extract according to any of claims 1 to 3, or a cosmetic composition according to any of claims 4 and 5 for increasing lysosomal activity in skin cells, preferably keratinocytes, in a subject, for cosmetic treatment and prevention of aged skin.

7. A non-therapeutic use of an extract according to any of claims 1 to 3, or a cosmetic composition according to any of claims 4 and 5 for increasing autophagy in skin cells, preferably keratinocytes, in a subject, for cosmetic treatment and prevention of aged skin.

8. A non-therapeutic use of an extract according to any of claims 1 to 3, or a cosmetic composition according to any of claims 4 and 5 for retarding the development or manifestation of aged skin, preferably photo-aged skin, in a subject.

9. The non-therapeutic use according to claims 6 to 8, wherein the aged skin is due to a decrease of lysosomal activity and/or autophagy in skin cells, preferably keratinocytes.

10. The non-therapeutic use according to any of claims 6 to 8, wherein the skin is facial or body skin, preferably aged skin, more preferably photo-aged skin, or is scalp skin.

## Patentansprüche

1. Extrakt aus *Galdieria sulphuraria,* umfassend einen Autophagie-Aktivator und/oder ein Eisenbindungsmittel oder einen Autophagie-Aktivator mit Eisenbindungseigenschaften, wobei der Extrakt Zelltrümmer enthält.

2. Extrakt nach Anspruch 1, bei dem es sich um ein wässriges Zellhomogenat oder -lysat oder eine Partikelfraktion davon oder ein Filtrat davon handelt.

3. Extrakt nach Anspruch 1 oder 2, der einer Trocknung, bevorzugt Gefriertrocknung unterzogen wurden.

4. Kosmetische Zusammensetzung, umfassend einen Extrakt nach einem der Ansprüche 1 bis 3.

5. Kosmetische Zusammensetzung nach Anspruch 4, die an eine topische Anwendung auf die Haut eines Individuums angepasst ist.

6. Nicht therapeutische Verwendung eines Extrakts nach einem der Ansprüche 1 bis 3 oder einer kosmetischen Zusammensetzung nach einem der Ansprüche 4 und 5 zur Steigerung lysosomaler Aktivität in Hautzellen, vorzugsweise Keratinozyten, bei einem Individuum, zur kosmetischen Behandlung und Vorbeugung gealterter Haut.

7. Nicht therapeutische Verwendung eines Extrakts nach einem der Ansprüche 1 bis 3 oder einer kosmetischen Zusammensetzung nach einem der Ansprüche 4 und 5 zur Steigerung von Autophagie in Hautzellen, vorzugsweise Keratinozyten, bei einem Individuum, zur kosmetischen Behandlung und Vorbeugung gealterter Haut.

8. Nicht therapeutische Verwendung eines Extrakts nach einem der Ansprüche 1 bis 3 oder einer kosmetischen Zusammensetzung nach einem der Ansprüche 4 und 5 zur Verzögerung der Entwicklung oder Manifestierung von gealterter Haut, vorzugsweise lichtgealterter Haut, bei einem Individuum.

9. Nicht therapeutische Verwendung nach Anspruch 6 bis 8, wobei die gealterte Haut auf eine Abnahme lysosomaler Aktivität und/oder von Autophagie in Hautzellen, vorzugsweise Keratinozyten, zurückzuführen ist.

10. Nicht therapeutische Verwendung nach Anspruch 6 bis 8, wobei es sich bei der Haut um Gesichts- oder Körperhaut, bevorzugt gealterte Haut, stärker bevorzugt lichtgealterte Haut, oder um Kopfhaut handelt.

## Revendications

1. Extrait de *Galdieria sulphuraria,* comprenant un activateur d'autophagie et/ou un agent de liaison du fer, ou un activateur d'autophagie possédant des propriétés de liaison du fer, l'extrait contenant des débris cellulaires.

2. Extrait selon la revendication 1, qui est un homogénat ou un lysat aqueux de cellules, ou une fraction particulaire correspondante, ou un filtrat correspondant.

3. Extrait selon la revendication 1 ou 2, qui a été soumis à un séchage, préférablement une lyophilisation.

4. Composition cosmétique comprenant un extrait selon l'une quelconque des revendications 1 à 3.

5. Composition cosmétique selon la revendication 4, qui est adaptée pour une application topique à la peau d'un sujet.

6. Utilisation non thérapeutique d'un extrait selon l'une quelconque des revendications 1 à 3, ou d'une composition cosmétique selon l'une quelconque des revendications 4 et 5 pour l'augmentation de l'activité lysosomale dans des cellules de la peau, préférablement des kératinocytes, chez un sujet, pour le traitement cosmétique et la prévention du vieillissement de la peau.

7. Utilisation non thérapeutique d'un extrait selon l'une quelconque des revendications 1 à 3, ou d'une composition cosmétique selon l'une quelconque des revendications 4 et 5 pour l'augmentation de l'autophagie dans des cellules de la peau, préférablement des kératinocytes, chez un sujet, pour le traitement cosmétique et la prévention du vieillissement de la peau.

8. Utilisation non thérapeutique d'un extrait selon l'une quelconque des revendications 1 à 3, ou d'une composition cosmétique selon l'une quelconque des revendications 4 et 5 pour le retardement du développement ou de la manifestation du vieillissement de la peau, préférablement du photovieillissement de la peau, chez un sujet.

9. Utilisation non thérapeutique selon les revendications 6 à 8, le vieillissement de la peau étant dû à une diminution de l'activité lysosomale et/ou de l'autophagie dans des cellules de la peau, préférablement des kératinocytes.

10. Utilisation non thérapeutique selon les revendications 6 à 8, la peau étant de la peau du visage ou du corps, préférablement de la peau vieillie, plus préférablement de la peau photo-vieillie, ou étant de la peau du cuir chevelu.
